(19) Europäisches Patentamt
European Patent Office
Office européen des brevets

(11) **EP 2 502 554 A1**

## (12) EUROPEAN PATENT APPLICATION
published in accordance with Art. 153(4) EPC

(43) Date of publication:
**26.09.2012 Bulletin 2012/39**

(21) Application number: **10831723.1**

(22) Date of filing: **08.09.2010**

(51) Int Cl.:
**A61B 5/02** (2006.01)     **A61B 5/026** (2006.01)

(86) International application number:
**PCT/KR2010/006080**

(87) International publication number:
**WO 2011/062356 (26.05.2011 Gazette 2011/21)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **17.11.2009 KR 20090110702**

(71) Applicant: **H3 System Co., Ltd.**
**Daejeon 305-340 (KR)**

(72) Inventors:
• **KIM, Min-Joon**
**Daejeon 305-729 (KR)**

• **KIM, Jung**
**Daejeon 305-762 (KR)**
• **HAN, Hyo-Nyoung**
**Daejeon 305-701 (KR)**
• **KIM, Young-Hwa**
**Daejeon 306-781 (KR)**

(74) Representative: **Harrison, Michael Robert**
**Harrison IP**
**Box Tree House**
**Northminster Business Park**
**Northfield Lane**
**York, Yorkshire Y026 6QU (GB)**

## (54) PHOTOPLETHYSMOGRAPHY APPARATUS

(57)     A photoplethysmography apparatus is provided. The photoplethysmography includes a plurality of light-emitting units spaced apart from each other and configured to emit light to a measurement part, a light-receiving unit disposed in the center of the light-emitting units and configured to detect transmitted or reflected light from the measurement part by the light-emitting units, a signal pre-processing unit configured to amplify and filter a measurement signal of the light-receiving unit, and a signal processing unit configured to extract a heart rate of a target person using an output signal of the pre-processing unit. The light-emitting units and the light-receiving unit provide a plurality of optical paths, and the light-receiving unit detects spatially averaged reflected or transmitted light at the measurement part.

Fig. 1

**Description**

BACKGROUND OF THE INVENTION

1. Field of the Invention

The present invention generally relates to heart rate monitors and, more particularly, to a photoplethysmography apparatus.

2. Description of the Related Art

Photoplethysmograph (PPG) is a kind of pulse wave measuring method. In the PPG, the amount of blood flowing through a blood vessel is measured using optical characteristics of biological tissues to understand heart rate activity states. A pulse wave has a pulsar waveform generated while blood is waved in a heart. The pulse wave may be measured through change in the amount of flowing blood (i.e., change in the volume of a blood vessel) which is caused by cardiac relaxation and contraction. PPG is a pulse wave measuring method using light. According to the PPG, an optical sensor detects and measures variation of optical characteristics such as reflection, absorption, and transmission ratios of biological tissues. Thus, a heart rate may be measured. The PPG has been widely used due to advantages such as noninvasive heart rate measurement, miniaturization, and convenience of use. In addition, the PPG facilitates developments of wearable biological signal sensors. Nonetheless, a photoplethysmograph sensor is disadvantageous in that a measurement signal is severely distorted when motion is accompanied.

SUMMARY OF THE INVENTION

Embodiments of the present invention provide a photoplethysmography apparatus with reduced motion artifact.

According to an aspect of the present invention, a photoplethysmography apparatus may include a plurality of light-emitting units spaced apart from each other and configured to emit light to a measurement part; a light-receiving unit disposed in the center of the light-emitting units and configured to detect transmitted or reflected light from the measurement part by the light-emitting units; a signal pre-processing unit configured to amplify and filter a measurement signal of the light-receiving unit; and a signal processing unit configured to extract a heart rate of a target person using an output signal of the pre-processing unit. The light-emitting units and the light-receiving unit may provide a plurality of optical paths, and the light-receiving unit may detect spatially averaged reflected or transmitted light at the measurement part.

In one embodiment of the present invention, the light-emitting units may be symmetrically arranged with respect to the light-receiving unit.

In one embodiment of the present invention, each of the light-emitting units may be a light-emitting diode (LED) configured to emit read or visible light.

In one embodiment of the present invention, the photoplethysmography apparatus may further include an acceleration sensor attached to the measurement part and configured to detect motion (acceleration of x, y, and z axes). The signal pre-processing unit may amplify and filter an output signal of the acceleration sensor, and the signal processing unit may compensate motion artifact caused by the motion using an output signal of the signal pre-processing unit to extract a heart rate of the target person.

According to another aspect of the present invention, a photoplethysmography apparatus may include light-receiving units spaced apart from each other and attached to a measurement part of a target person and configured to detect reflected or transmitted light from the measurement part; a light-emitting unit disposed in the center of the light-receiving units and attached to the measurement part and configured to provide output light to the measurement part; an acceleration sensor attached to the measurement part and configured to detect motion (acceleration of x, y, and z axes); a signal pre-processing unit configured to amplify and filter output signals of the light-receiving units and the acceleration sensor; and a signal processing unit configured to compensate dynamic disturbance caused by the acceleration using the output signal of the signal pre-processing unit extract a heart rate of the target person. The signal processing unit may process output signals of the light-receiving units after averaging the output signals.

In one embodiment of the present invention, the light-receiving units may be symmetrically arranged with respect to the light-emitting unit.

According to further another aspect of the present invention, a photoplethysmography apparatus may include a light-emitting unit; a first reflecting unit configured to reflect output light of the light-emitting unit in one direction or to one side; a second reflecting unit disposed on the center of the first reflecting unit and configured to reflect of output light of the light-emitting unit or re-reflect the light reflected from the first reflecting unit and provide the re-reflected light to the first reflecting unit; and a light-receiving unit mounted on the second reflecting unit and attached to a measurement part of a target person. The first reflecting unit and the second reflecting unit may provide a plurality of symmetrical optical paths between the light-emitting unit and the light-receiving around the measurement part.

In one embodiment of the present invention, the first reflecting unit and the second reflecting unit may illuminate the measurement part at a plurality of positions.

BRIEF DESCRIPTION OF THE DRAWINGS

The present invention will become more apparent in view of the attached drawings and accompanying detailed description. The embodiments depicted therein are provided by way of example, not by way of limitation, wherein like reference numerals refer to the same or similar elements. The drawings are not necessarily to scale, emphasis instead being placed upon illustrating aspects of the present invention.

FIG. 1 illustrates a photoplethysmography apparatus according to one embodiment of the present invention.

FIG. 2 illustrates an adaptive filter of a signal processing unit in a photoplethysmography apparatus according to one embodiment of the present invention.

FIG. 3 illustrates a photoplethysmography apparatus according to another embodiment of the present invention.

FIGS. 4 and 5 illustrate a photoplethysmography apparatus according to further another embodiment of the present invention.

FIGS. 6 and 7 illustrate a photoplethysmography apparatus according to other embodiments of the present invention.

FIGS. 8 and 9 illustrate a photoplethysmography apparatus according to further other embodiments of the present invention.

FIG. 10 is a graphic diagram illustrating a result measured by a conventional photoplethysmography apparatus and a result measured by an electrocardiogram-based heart rate monitor.

FIG. 11 is a graphic diagram illustrating a result measured by a photoplethysmography apparatus according to one embodiment of the present invention and a result measured by an electrocardiogram-based heart rate monitor.

DETAILED DESCRIPTION OF EMBODIMENTS

Causes of motion artifact generated by motion may be examined as a physiological structural problem and a device structural problem. For example, if looking into the direction on a wrist, an x-axial direction matches a direction of aorta radialis blood vessel passing through the wrist, and motion in the x-axial direction may have an influence on change in the amount and flow rate of blood flowing through a blood vessel. Accordingly, the motion in the x-axial direction has an effect on the volume of the blood vessel and has an direct effect thereon through a photoplethysmography apparatus. In addition, noise in the z-axial direction may be examined as a device structural affect. The motion in the z-axial direction applies a pressure to the skin due to mass and inertia of a photoplethysmography apparatus. Since the pressure leads to change of the skin and blood vessel, motion artifact may be generated.

A photoplethysmography apparatus according to one embodiment of the present invention provides a plurality of optical paths between a light-receiving unit and light-emitting units that are spatially apart from each other at the skin. Thus, a spatially averaged optical signal may be detected to minimize motion artifact caused by a local position. As a result, the photoplethysmography apparatus may decrease generation of motion artifact caused by motion to extract an accurate heart rate.

Preferred embodiments of the present invention will be described below in more detail with reference to the accompanying drawings. The present invention may, however, be embodied in different forms and should not be constructed as limited to the embodiments set forth herein. Rather, these embodiments are provided so that this disclosure will be thorough and complete, and will fully convey the scope of the invention to those skilled in the art. Like numerals refer to like elements throughout the specification.

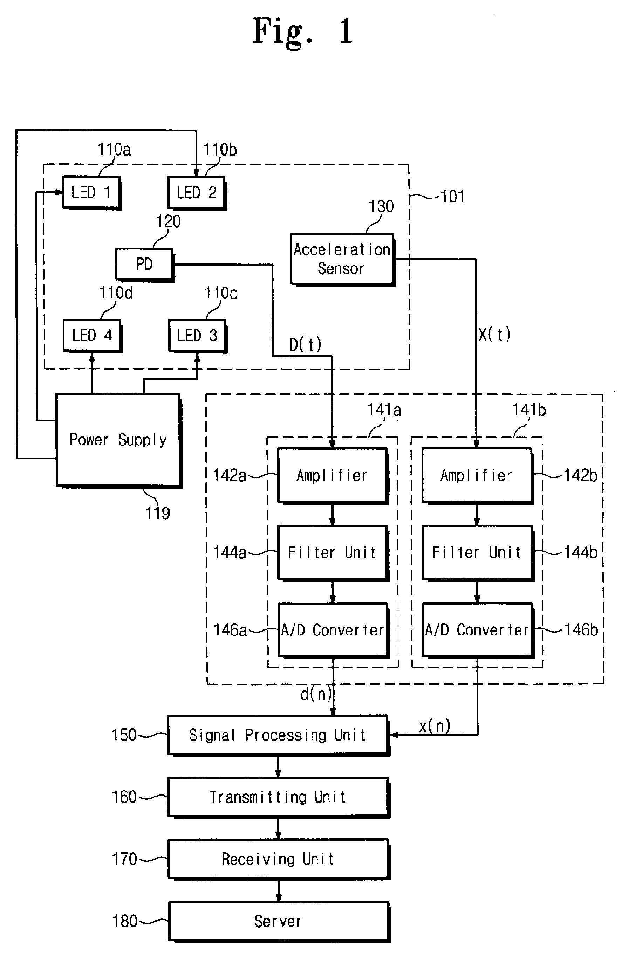

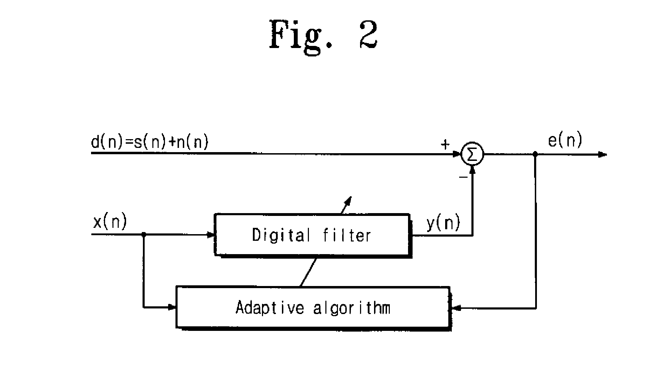

FIG. 1 illustrates a photoplethysmography apparatus according to one embodiment of the present invention, and FIG. 2 illustrates an adaptive filter of a signal processing unit in a photoplethysmography apparatus according to one embodiment of the present invention.

Referring to FIG. 1, the photoplethysmography apparatus includes a plurality of light-emitting units 110a~110d spaced apart from each other and configured to emit light to a measurement part, a light-receiving unit 120 disposed in the center of the light-emitting units 110a~110d and configured to detect transmitted or reflected light from the measurement part by the light-emitting units 110a~1110d, a signal pre-processing unit 150 configured to amplify and filter a measurement signal D(t) of the light-receiving unit 120, and a signal processing unit 150 configured to extract a heart rate of a target person using signals d(n) and x(n) of the pre-processing unit 140. The light-emitting units 110a~110d and the light-receiving unit 120 provide a plurality of optical paths. The light-receiving unit 120 detects spatially averaged reflected or transmitted light at the measurement part.

The photoplethysmography apparatus may detect change in the amount of blood flowing through a blood vessel of the measurement part of the target person. The measurement part of the target person may be ear, finger, toe, neck, wrist or forehead. The measurement part of the target person may be defined as a region surrounded by the light-emitting units 110a~110d.

The light-emitting units 110a~110d may emit infrared or visible light. The light-emitting units 110a~110d may include a self light-emitting element or a light-emitting element using a florescent substance. Specifically, each of the light-emitting units 110a~110d may be an infrared light emitting diode (infrared LED), a blue LED, a red LED or a green LED. The light-emitting units 110a~110d are symmetrically arranged with respect to the light-receiving unit. The light-emitting units 110a~110d may receive a power through one power supply 119. The power supply 119 may be a battery or a DC power supply.

The light-receiving unit 120 may be disposed in the center of the light-emitting units 110a~110d. The light-receiving unit 120 may receive reflected or transmitted light of the light-emitting units 110a~110d. The light-receiving unit 120 may further include an optical filter. The light-receiving unit 120 may include at least one selected from the group consisting of a photodiode (PD), a charge-coupled device (CCD), and a complementary image sensor (CIS). The light-receiving unit 120 may further include a light-collecting unit (not shown) configured to collect the transmitted or reflected light of the light-emitting units 110a~110d. In the case that the light-receiving unit 120 includes the light-collecting unit, the light-collecting unit may be disposed in the center of the light-emitting units 110a~110d. The light-receiving unit 120 may output an analog measurement signal D(t).

When one light-emitting unit is used, the light-emitting unit and the light-receiving unit provide one optical path. Accordingly, when there is a dynamic motion, an optical path from the light-emitting unit to the light-receiving unit and intensity may be changed. A pressure of a local skin generated by the dynamic motion may lead to change of skin and blood vessel to cause motion artifact.

In order to reduce signal distortion caused by dynamic motion (i.e., motion artifact), a photoplethysmography apparatus according to one embodiment of the present invention provides a plurality of optical paths between the plurality of light-emitting units 110a~110d and the light-receiving unit 120. Thus, the light-receiving unit 120 may obtain spatially averaged transmitted or reflected light even when there is dynamic motion. The motion artifact may be spatially averaged to be reduced.

Additionally, specific-directional motion may have an effect on the amount and flow rate of blood flowing through a blood vessel. The specific-directional motion may be corrected by means of an acceleration sensor 130.

The acceleration sensor 130 may be a tri-axial acceleration sensor. The acceleration sensor 130 may output an analog acceleration signal X(t) in x, y, and z-axial directions. The acceleration sensor 130 may provide information about dynamic motion of the measurement part. The acceleration sensor 130 may be disposed such that its central axis matches the central axis of the light-receiving unit 120. A sensor unit 101 may include the light-emitting units 110a~110d, the light-receiving unit 120, and the acceleration sensor 130. The sensor unit 101 may be packaged in one body.

The signal pre-processing unit 140 may include a first signal pre-processing unit 141a and a second signal pre-processing unit 141b. The first signal pre-processing unit 141a may receive and process the measurement signal D(t) of the light-receiving unit 120. The second signal pre-processing unit 141b may receive and process the acceleration signal X(t) of the acceleration sensor 130. The acceleration sensor 130 may output an x-axial acceleration signal, a y-axial acceleration signal, and a z-axial acceleration signal. Accordingly, the second signal pre-processing unit 141b may output three digital acceleration signal x(n) with three channels.

The first signal pre-processing unit 141a may include at least one selected from the group consisting of an amplifier 142a, a filter unit 144a, and an A/D converter 146a. The second signal pre-processing unit 141b may include at least one selected from the group consisting of an amplifier 142b, a filter unit 144b, and an A/D converter 146b. The amplifier 142a may amplify the measurement signal D(t) of the light-receiving unit 120. The filter unit 144a may include at least one selected from the group consisting of a bandpass filter, a lowpass filter, and a highpass filter which selectively pass frequency components of a person's heart rate. The filter unit 144a may be comprised of a passive element or an active element. A cutoff frequency of the lowpass filter may be about 5 Hz. A cutoff frequency of the highpass filter may be about 0.5 Hz. The A/D converter 146a may convert an analog signal to a digital signal to output a digital measurement signal d(n). The A/D converter 146b may convert an analog signal to a digital signal to output a digital acceleration signal x(n). A driving clock frequency of the A/D converters 146a and 146b may be about 200 Hz.

The signal processing unit 150 may receive the digital measurement signal d(n) and the digital acceleration signal x(n) of the signal pre-processing unit 140. The signal processing unit 150 may include a digital signal processor (DSP) or a microprocessor. The signal processing unit 150 may adaptive filter algorithm to remove motion artifact.

Referring to FIG. 2, the digital measurement signal d(n) may include a pulse wave signal S(n) and dynamic noise associated with motion (i.e., motion artifact) n(n). The digital acceleration signal x(n) may have a direct correlation to the motion artifact n(n). The pulse wave signal S(n) may be obtained by removing the motion artifact from the digital measurement signal d(n). Accordingly, an estimate y(n) of the motion artifact may be provided using the digital acceleration signal x(n). An estimate e(n) of the pulse wave signal may be obtained by subtracting the estimate y(n) of the motion artifact from the digital measurement signal d(n). The estimate y(n) of the motion artifact may be obtained through the acceleration signal x(n) and a digital filter having a filter coefficient w(n), as follow:

[Equation 1]

$$y(n) = \sum_{l=0}^{l-1} w_l(n) x(n-l)$$

The filter coefficient w(n) may be optimized using the digital acceleration signal x(n) and the estimate e(n) of the pulse wave signal by an adaptive filter. The adaptive filter may use least means square (LMS) algorithm. A heart rate may be extracted using the estimate e(n) of the pulse wave signal according to time.

Returning to FIG. 1, a transmitting unit 160 may provide a heart rate of the signal processing unit 150 through wired or wireless communication. A receiving unit 170 may receive the heart rate through wired/wireless communication with the transmitting unit 160. Information of the receiving unit 170 may be stored in a server 180.

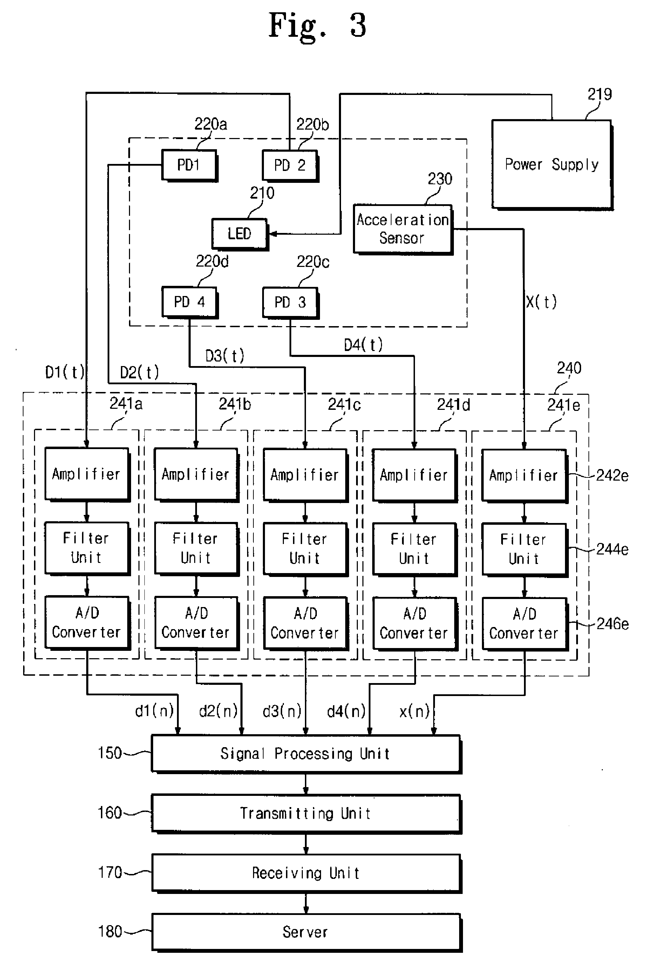

FIG. 3 illustrates a photoplethysmography apparatus according to another embodiment of the present invention. In FIG. 3, duplicate explanations as those described in FIG. 1 will be omitted.

Referring to FIG. 3, the photoplethysmography apparatus includes light-receiving units 220a~220d spaced apart from each other and attached to a measurement part of a target person and configured to detect reflected or transmitted light from the measurement part, a light-emitting unit 210 disposed in the center of the light-receiving units 220a~220d and attached to the measurement part and configured to provide output light to the measurement part, an acceleration sensor 230 attached to the measurement part and configured to detect acceleration of x, y, and z axes, a signal pre-processing unit 240 configured to amplify and filter output signals of the light-receiving units 220a~220d and the acceleration sensor 230, and a signal processing unit 150 configured to compensate dynamic disturbance caused by the acceleration using the output signal of the signal pre-processing unit 240 to extract a heart rate of the target person. The signal processing unit 150 processes output signals of the light-receiving units 220a~220d after averaging the output signals.

The light-emitting unit 210 may be a light-emitting diode (LED). The light-emitting unit 210 may be disposed in the center of the measurement part of the target person. A power supply 219 may supply a direct current (DC) power to the light-emitting unit 210.

The light-receiving units 220a~220d may be symmetrically disposed around the light-emitting unit 210. Each of the light-receiving units 220a~220d may be a photodiode (PD). The light-emitting unit 210 and the light-receiving units 220a~220d may provide a plurality of optical paths. Thus, the output signals of the light-receiving units 220a~220d may be averaged to minimize motion artifact.

The acceleration sensor 230 may be disposed such that its central axis matches the central axis of the light-receiving unit 210. The acceleration sensor 230 may be a tri-axial acceleration sensor.

The signal pre-processing unit 240 may include an amplifier, a filter unit, and an A/D converter. The signal pre-processing unit 240 may include first to fifth signal pre-processing units 241a~241e. The first to fourth signal pre-processing units 241a~241d may amplify and filter measurement signals D1(t), D2(t), D3(t), and D4(t) of the light-receiving units 220a~220d. The A/D converter may convert an analog signal to a digital signal to digital measurement signals d1(n), d2(n), d3(n), and d4(n). The fifth signal pre-processing unit 241e may receive an acceleration signal X(t) and amplify and filter the received signal X(t). Also the fifth signal pre-processing unit 241e may convert the amplified and filtered signal X(t) to a digital signal to output a digital acceleration signal x(n).

The signal processing unit 250 may sum and average the digital measurement signals d1(n), d2(n), d3(n), and d4(n). The averaged digital measurement signal and the digital acceleration signal x(n) may be provided to adaptive filter algorithm to remove motion artifact.

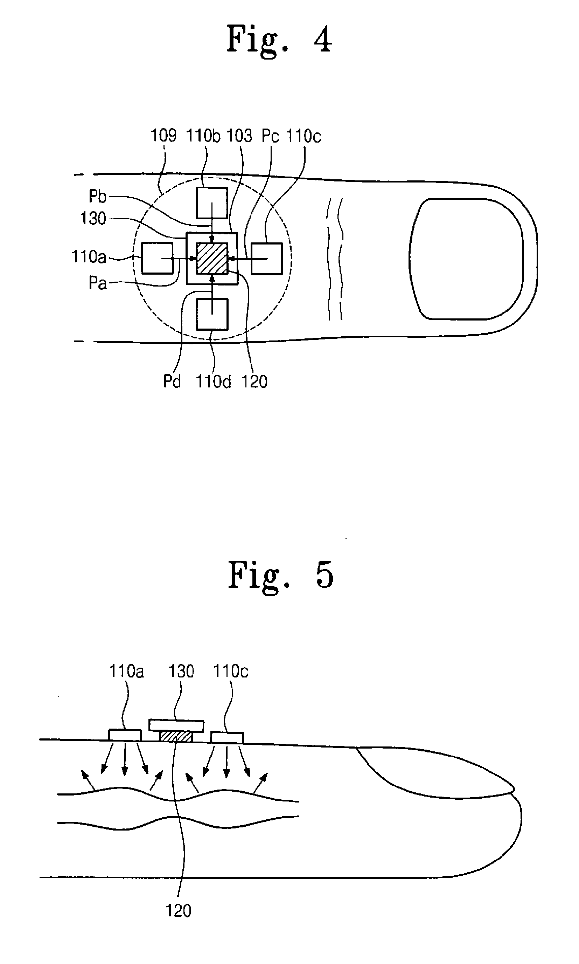

FIGS. 4 and 5 illustrate a photoplethysmography apparatus according to further another embodiment of the present invention. FIG. 5 is a side view of FIG. 4.

Referring to FIGS. 4 and 5, the photoplethysmography apparatus includes a plurality of light-emitting units 110a, 110b, 110c, and 110d spaced apart from each other and configured to emit light to a measurement part 109 and a light-receiving unit 120 disposed in the center of the light-emitting units 110a, 110b, 110c, and 110d and configured to detect transmitted or reflected light from the measurement part by the light-emitting units 110a, 110b, 110c, and 110d. The light-emitting units 110a, 110b, 110c, and 110d and the light-receiving unit 120 provide a plurality of optical paths Pa, Pb, Pc, and Pd. The light-receiving unit 120 detects spatially averaged light reflected from or transmitted to the measurement part 109. An acceleration sensor 130 may be disposed on the light-receiving unit 120. The light-emitting units 110a, 110b, 110c, and 110d may have the same light-emitting efficiency. The light-emitting units 110a, 110b, 110c, and 110d may be fabricated to have a wide view angel. The light-emitting units 110a, 110b, 110c, and 110d may be arranged in the form of a cross.

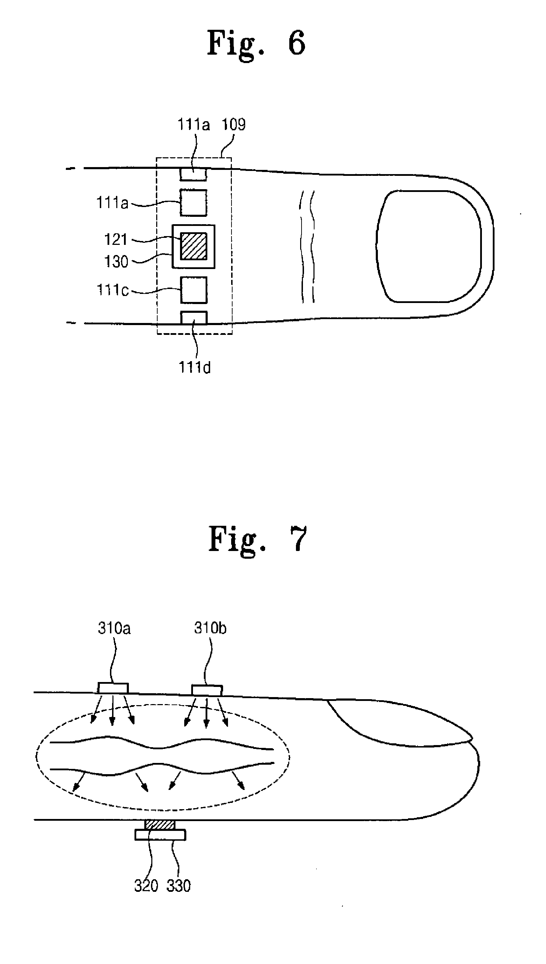

FIGS. 6 and 7 illustrate a photoplethysmography apparatus according to other embodiments of the present invention.

Referring to FIG. 6, light-emitting units 111a~111d may be arranged in a line. A light-receiving unit 121 may be disposed in the center of the light-emitting units 111a~111d. The light-emitting units 111a~111d may provide a measurement region 109. The light-receiving unit 121 may receive reflected light or transmitted light of output light of the light-emitting units 111a~111d. An acceleration sensor 130 may be disposed on the light-receiving unit 121.

Referring to FIG. 7, light-emitting units 310a and 310b may be disposed on one surface of a measurement part, and a light-receiving unit 320 may be disposed on the other surface thereof. The light-emitting units 310a and 310b may be disposed symmetrically with respect to the light-receiving unit 320. The light-receiving unit 320 may receive transmitted light of output light of the light-emitting units 310a and 310b. The light-receiving unit 320 may receive an averaged optical signal by a plurality of optical paths. An acceleration sensor 330 may be disposed below the light-receiving unit 320.

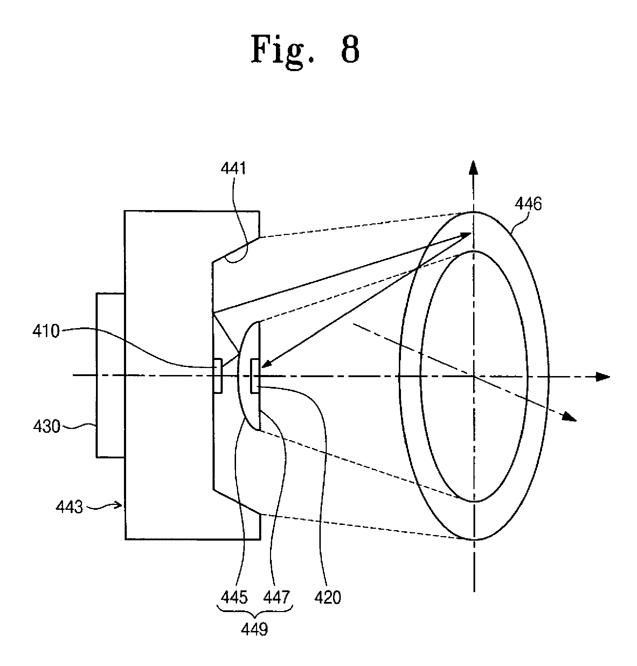

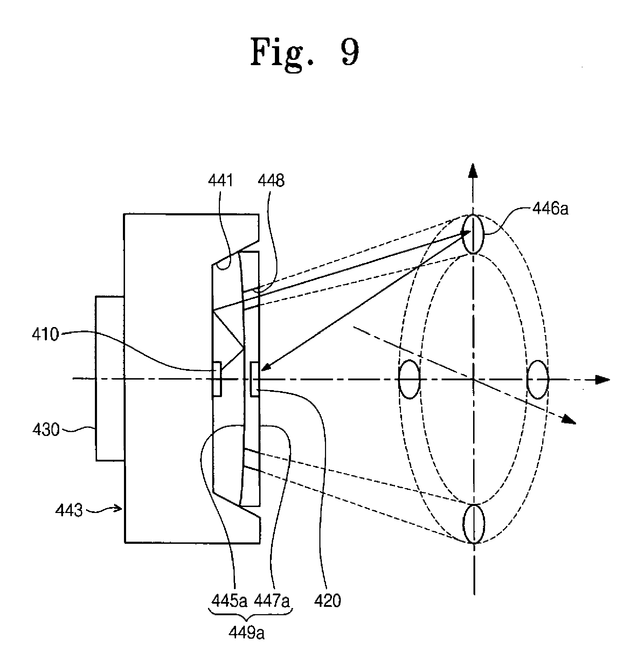

FIGS. 8 and 9 illustrate a photoplethysmography apparatus according to further other embodiments of the present invention.

Referring to FIG. 8, a photoplethysmography apparatus includes a light-emitting unit 410, a first reflecting unit 441 configured to reflect output light of the light-emitting unit 410 in one direction or to one side, a second reflecting unit 449 disposed on the center of the first reflecting unit 441 and configured to reflect of output light of the light-emitting unit 410 or re-reflect the light reflected from the first reflecting unit 441 and provide the re-reflected light to the first reflecting unit 441, and a light-receiving unit 420 mounted on the second reflecting unit 449 and attached to a measurement part of a target person. The first reflecting unit 441 and the second reflecting unit 449 provide a plurality of symmetrical optical paths between the light-emitting unit 410 and the light-receiving 420 around the measurement part.

The light-emitting unit 410 may be a light-emitting diode (LED). The light-emitting unit 410 may be mounted on the center of a frame 443 including the first reflecting unit 441. The first reflecting unit 441 may be a reflecting cup. The first reflecting unit 441 may be fabricated such that the output light of the light-emitting unit 410 has a uniform illumination to one side. The first reflecting unit 441 may be coated with a metal. The first reflecting unit 441 may be in the shape of a tapered cup.

The second reflecting unit 449 may be disposed on the central axis of the first reflecting unit 441. The second reflecting unit 449 may have a hemispherical shape including a hemispherical surface 445 and a flat surface 447. The hemispherical surface 445 may be disposed opposite to the first reflecting unit 441. The first reflecting unit 441 and the second reflecting unit 449 may illuminate to form ring (446) shaped pattern on the measurement part.

The light-receiving unit 420 may be disposed on the flat surface 447 of the second reflecting unit 449. The light emitted from the light-emitting unit 410 may reach the light-receiving unit 420 after being reflected from or transmitted to the measurement part. The optical path between the light-emitting unit 410 and the light-receiving unit 420 may be a plurality of optical paths. An acceleration sensor 430 may be mounted on the frame 443.

Referring to FIG. 9, a photoplethysmography apparatus includes a light-emitting unit 410, a first reflecting unit 441 configured to output light of the light-emitting unit 410 in one direction or to one side, a second reflecting unit 449 disposed on the center of the first reflecting unit 441 and configured to reflect of output light of the light-emitting unit 410 or re-reflect the light reflected from the first reflecting unit 441 and provide the re-reflected light to the first reflecting unit 441, and a light-receiving unit 420 mounted on the second reflecting unit 449 and attached to a measurement part of a target person. The first reflecting unit 441 and the second reflecting unit 449 may a plurality of symmetrical optical paths between the light-emitting unit 410 and the light-receiving unit 420 around the measurement part.

The light-emitting unit 410 may be a light-emitting diode (LED). The light-emitting unit 410 may be mounted on the center of a frame 443 including the first reflecting unit 441. The first reflecting unit 441 may be a reflecting cup. The first reflecting unit 441 may be fabricated such that the output light of the first light-emitting unit 410 has a uniform illumination to one side. The first reflecting unit 441 may be coated with a metal. The first reflecting unit 441 may be in the shape of a tapered cup.

The second reflecting unit 449a may be disposed on the central axis of the first reflecting unit 441. The second reflecting unit 449a may include a reflecting surface 445a and a flat surface 447a. The second reflecting unit 449a may include a plurality of through-holes 448. Light passing through the through-holes 448 may be regularly emitted to the measurement part.

The light-receiving unit 420 may be disposed on the flat surface 447 of the second reflecting unit 449. The light emitted from the light-emitting unit 410 may reach the light-receiving unit 420 after being reflected from or transmitted to the measurement part. The light-emitting unit 410 and the light-receiving unit 420 may provide a plurality of optical paths. An acceleration sensor 430 may be mounted on the frame 443.

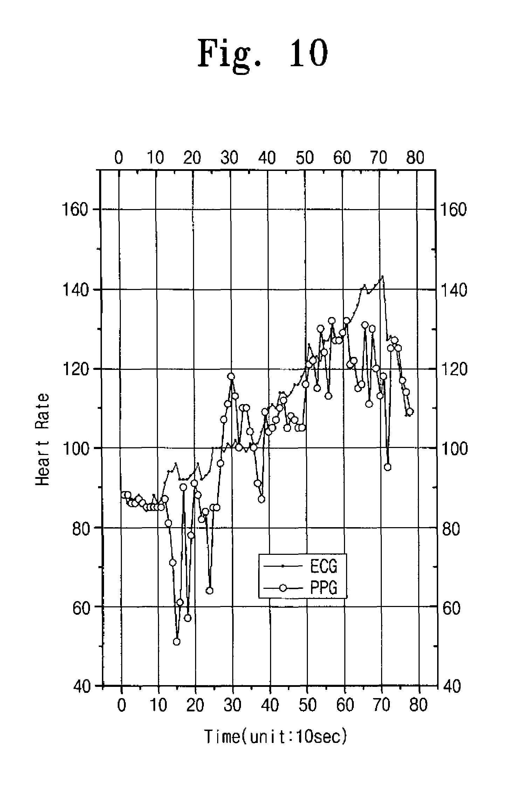

FIG. 10 is a graphic diagram illustrating a result measured by a conventional photoplethysmography apparatus and a result measured by an electrocardiogram-based heart rate monitor.

In FIG. 10, the graph shows a result of a photoplethysmography apparatus using one light-emitting unit and one light-receiving unit and a result of an electrocardiogram (hereinafter referred to as "ECG") based heart rate monitor.

The result of the ECG-based heart rate monitor provides a reference pulse signal. ECG used as a reference signal of heart rate was measured in a three-channel electrode manner. The test was conducted by attaching the ECG-based heart rate monitor to the chest which is less affected by motion artifact. The result of the photoplethysmography apparatus was measured at the finger.

The test was conducted on a treadmill. In the graph, the x-axis represents time (unit: 10 seconds). The treadmill continues to run through an interval of pause state (2 minutes 20 seconds), an interval of 3 km/hour (2 minutes 20 seconds), an interval of 5 km/hour (2 minutes 20 seconds), an interval of 7 km/hour (2 minutes 20 seconds), an interval of 10 km/hour (2 minutes 20 seconds), and an interval of pause and rest (1 minute 10 seconds).

In the interval of pause state, a target person's photoplethysmograph (PPG) measured by the photoplethysmography apparatus using one light-emitting unit and one light-receiving unit almost matches a result of the ECG-based heart rate monitor. However, if the target person starts exercise, the result of the photoplethysmography apparatus becomes different from that of the ECG-based heart rate monitor.

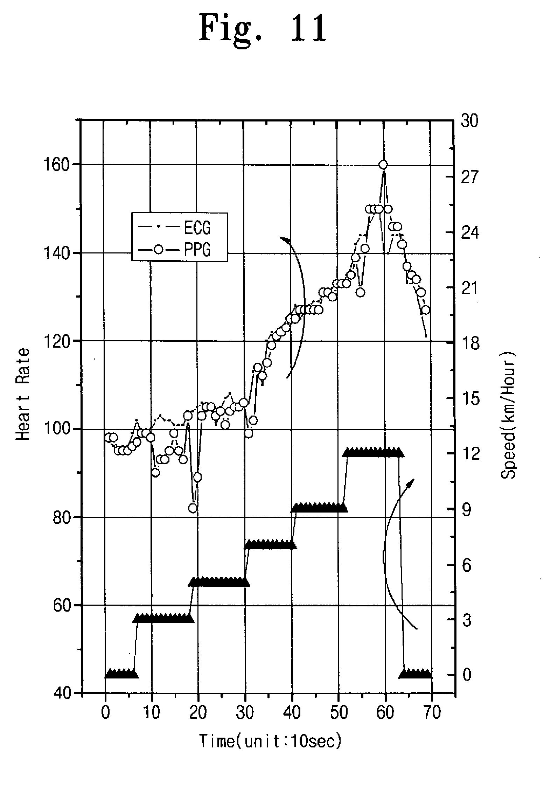

FIG. 11 is a graphic diagram illustrating a result measured by a photoplethysmography apparatus according to one embodiment of the present invention and a result measured by an electrocardiogram-based heart rate monitor. The target person in FIG. 9B and the target person in FIG. 9A are identical to each other.

Referring to FIG. 11, the photoplethysmography apparatus includes fourth light-emitting units and one light-receiving unit. The light-emitting unit employs an infrared light-emitting diode (LED), and the light-receiving unit employs a photodiode (PD).

The result measured by the ECG-based heart rate monitor provides a reference pulse signal. ECG used as a reference signal of heart rate was measured in a three-channel electrode manner. The test was conducted by attaching the ECG-based heart rate monitor to the chest which is less affected by motion artifact. The result of the photoplethysmography apparatus was measured at the finger.

The result of the ECG-based heart rate monitor provides a reference pulse signal. The test was conducted on a treadmill. In the graph, the x-axis represents time (unit: 10 seconds). The treadmill continues to run through an interval of pause state (1 minute), an interval of 3 km/ hour (2 minutes), an interval of 5 km/hour (2 minutes), an interval of 7 km/hour (1 minutes 40 seconds), an interval of 9 km/hour (1 minute 50 seconds), an interval of 12 km/hour (2 minutes), and an interval of pause and rest (1 minute).

In not only the interval of pause state but also the interval of 12 km/h, a target person's heart rate measured by the photoplethysmography apparatus almost matches a rate of the ECG-based heart rate monitor. Accordingly, the target person's heart rate was accurately measured even when the target person is in any situation.

According to the embodiments of the present invention described above, a photoplethysmography apparatus having a plurality of spatially optical paths exhibits strong characteristics against motion artifact. Thus, a heart rate can be accurately measured even in conventional excise situations where it is difficult to accurately measure a heart rate.

Although the present invention has been described in connection with the embodiment of the present invention illustrated in the accompanying drawings, it is not limited thereto. It will be apparent to those skilled in the art that various substitutions, modifications and changes may be made without departing from the scope and spirit of the present invention.

**Claims**

1. A photoplethysmography apparatus:

    a plurality of light-emitting units spaced apart from each other and configured to emit light to a measurement part;
    a light-receiving unit disposed in the center of the light-emitting units and configured to detect transmitted or reflected light from the measurement part by the light-emitting units;
    a signal pre-processing unit configured to amplify and filter a measurement signal of the light-receiving unit; and
    a signal processing unit configured to extract a heart rate of a target person using an output signal of the pre-processing unit,
    wherein the light-emitting units and the light-receiving unit provide a plurality of optical paths, and the light-receiving unit detects spatially averaged reflected or transmitted light at the measurement part.

2. The photoplethysmography apparatus of claim 1, wherein the light-emitting units are symmetrically arranged with respect to the light-receiving unit.

3. The photoplethysmography apparatus of claim 1, wherein each of the light-emitting units is a light-emitting diode (LED) configured to emit read or visible light.

4. The photoplethysmography apparatus of claim 1, further comprising:

    an acceleration sensor attached to the measurement part and configured to detect motion,
    wherein the signal pre-processing unit amplifies and filters an output signal of the acceleration sensor, and
    wherein the signal processing unit compensates motion artifact caused by the motion using an output signal of the signal pre-processing unit to extract a heart rate of the target person.

5. A photoplethysmography apparatus comprising:

    light-receiving units spaced apart from each other and attached to a measurement part of a target person and configured to detect reflected or transmitted light from the measurement part;
    a light-emitting unit disposed in the center of the light-receiving units and attached to the measurement part and configured to provide output light to the measurement part;
    an acceleration sensor attached to the measurement part and configured to detect motion;
    a signal pre-processing unit configured to amplify and filter output signals of the light-receiving units and the acceleration sensor; and
    a signal processing unit configured to compensate dynamic disturbance caused by the acceleration using the output signal of the signal pre-processing unit extract a heart rate of the target person,
    wherein the signal processing unit processes output signals of the light-receiving units after averaging the output signals.

6. The photoplethysmography apparatus of claim 5, wherein the light-receiving units are symmetrically arranged with respect to the light-emitting unit.

7. A photoplethysmography apparatus comprising:

> a light-emitting unit;
> a first reflecting unit configured to reflect output light of the light-emitting unit in one direction or to one side;
> a second reflecting unit disposed on the center of the first reflecting unit and configured to reflect of output light of the light-emitting unit or re-reflect the light reflected from the first reflecting unit and provide the re-reflected light to the first reflecting unit; and
> a light-receiving unit mounted on the second reflecting unit and attached to a measurement part of a target person,
> wherein the first reflecting unit and the second reflecting unit provide a plurality of symmetrical optical paths between the light-emitting unit and the light-receiving around the measurement part.

8. The photoplethysmography apparatus of claim 7, wherein the first reflecting unit and the second reflecting unit illuminate the measurement part at a plurality of positions.

# Fig. 1

# Fig. 2

d(n)=s(n)+n(n) ————————————————————————— + (Σ) —— e(n) ——►

x(n) ——————► | Digital filter | y(n)

| Adaptive algorithm |

# Fig. 3

# Fig. 4

109 110b 103 Pc 110c

Pb
130
110a
Pa

Pd 110d 120

# Fig. 5

110a   130   110c

120

# Fig. 6

# Fig. 7

# Fig. 8

# Fig. 9

# Fig. 10

# Fig. 11

## INTERNATIONAL SEARCH REPORT

| International application No. |
| --- |
| **PCT/KR2010/006080** |

**A. CLASSIFICATION OF SUBJECT MATTER**

*A61B 5/02(2006.01)i, A61B 5/026(2006.01)i*

According to International Patent Classification (IPC) or to both national classification and IPC

**B. FIELDS SEARCHED**

Minimum documentation searched (classification system followed by classification symbols)
A61B 5/02; A61B 5/026; A61B 5/11; A61B 5/1455

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: a plurality of, light emission, light reception, acceleration sensor

**C. DOCUMENTS CONSIDERED TO BE RELEVANT**

| Category* | Citation of document, with indication, where appropriate, of the relevant passages | Relevant to claim No. |
| --- | --- | --- |
| A | KR 10-0455289 B1 (SAMSUNG ELECTRONICS CO., LTD.) 08 November 2004<br>See abstract, claim 6, figures 1-4 | 1-8 |
| A | JP 2008-212258 A (FUJI XEROX CO LTD) 18 September 2008<br>See abstract, claim 1, figures 1, 2, 5 | 1-8 |
| A | JP 2009-034427 A (KONICA MINOLTA SENSING INC) 19 February 2009<br>See abstract, claim 1, figure 1 | 1-8 |

☐ Further documents are listed in the continuation of Box C.　　☒ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br><br>29 MARCH 2011 (29.03.2011) | Date of mailing of the international search report<br><br>**31 MARCH 2011 (31.03.2011)** |
| Name and mailing address of the ISA/KR<br>　Korean Intellectual Property Office<br>　Government Complex-Daejeon, 139 Seonsa-ro, Daejeon 302-701,<br>　Republic of Korea<br>Facsimile No. 82-42-472-7140 | Authorized officer<br><br><br>Telephone No. |

Form PCT/ISA/210 (second sheet) (July 2009)

INTERNATIONAL SEARCH REPORT
Information on patent family members

International application No.

**PCT/KR2010/006080**

| Patent document cited in search report | Publication date | Patent family member | Publication date |
|---|---|---|---|
| KR 10-0455289 B1 | 08.11.2004 | CN 100335001 C0 | 05.09.2007 |
| | | EP 1344488 A2 | 17.09.2003 |
| | | EP 1344488 A3 | 15.09.2004 |
| | | EP 1834577 A2 | 19.09.2007 |
| | | EP 1834577 A3 | 03.10.2007 |
| | | JP 2003-265446 A | 24.09.2003 |
| | | US 2003-0236647 A1 | 25.12.2003 |
| | | US 6990426 B2 | 24.01.2006 |
| JP 2008-212258 A | 18.09.2008 | JP 2008-212258 A | 18.09.2008 |
| JP 2009-034427 A | 19.02.2009 | EP 2022394 A1 | 11.02.2009 |
| | | US 2009-0036762 A1 | 05.02.2009 |

Form PCT/ISA/210 (patent family annex) (July 2009)